(19) Europäisches Patentamt / European Patent Office / Office européen des brevets

(11) EP 3 184 039 A1

(12) **EUROPEAN PATENT APPLICATION**
published in accordance with Art. 153(4) EPC

(43) Date of publication:
28.06.2017 Bulletin 2017/26

(21) Application number: 15833007.6

(22) Date of filing: 21.07.2015

(51) Int Cl.:
*A61B 5/02* (2006.01)       *H04W 4/00* (2009.01)

(86) International application number:
PCT/IB2015/055526

(87) International publication number:
WO 2016/027179 (25.02.2016 Gazette 2016/08)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(30) Priority: 18.08.2014 MX MX14009943

(71) Applicant: Grupo P.I. Mabe S.A. de C.V.
Colonia Loma, Puebla 72230 (MX)

(72) Inventors:
• GONZÁLEZ MARTÍNEZ, Raúl
72230 Puebla (MX)
• SÁNCHEZ FERNÁNDEZ, Lucía Del Carmen
72230 Puebla (MX)
• CANALES ESPINOSA DE LOS MONTEROS, Carlos
72230 Puebla (MX)
• CORONA CARLOS, Alberto
72230 Puebla (MX)

(74) Representative: Gil-Vega, Victor
Corazón de Maria, 6
28002 Madrid (ES)

(54) **SYSTEM FOR SENSING VITAL SIGNS ASSOCIATED WITH THE USE OF DISPOSABLE ABSORBENT ARTICLES**

(57)    The present invention protects a system for monitoring the vital signs of a user of a disposable absorbent article that comprises a sensor device, a bi-dimensional code and a receiver, such that the sensor device is placed in contact with the user and the system is triggered by the reading of the bi-dimensional code through the receiver, such that the bi-dimensional code is place inside the packaging of the disposable absorbent article.

Fig. 1

EP 3 184 039 A1

**Description**

**Background of the Invention**

In recent years, an infinitude of proposals related to the use of computer technology associated with the use of disposable absorbent articles have been developed; such that through a sensor, any change in state of the article is detected, such as the presence of moisture and/or fecal matter, changes in pH, changes in temperature, infections, etc., the information detected by the sensor is transmitted to a receiver and said receiver sends a signal that may be transmitted and received in a mobile device, alerting the bearer of said device that such a change has occurred. In this way, the caregiver is alerted and knows when it is time to remove the article for replacing it with a new one and/or if the user of the article has undergone any change in his general health status.

The caregivers of a baby, particularly the parents, would have peace of mind and confidence to have the possibility of knowing and monitoring the vital signs, such as blood pressure, pulse, breathing rate and temperature of the baby at all times, receiving information about said vital signs on their mobile device wherever they are and continuously, as well as receiving an alarm signal in case these vital signs exceed the limits. The above also applies to the caregivers of disabled adults.

The present invention refers to a system for detecting the vital signs of a user of a disposable absorbent article, such as a diaper or disposable pants, through a device that is in contact with the user that is activated by a code or trigger mechanism associated with the article. This device detects the vital signs and sends a signal to a receiver. The receiver may be a mobile device or may receive the signal and send it to a mobile device.

As mentioned, there is a large amount of developments of sensors which are inserted inside the structure of the diaper and that are triggered by the release of urine, sending signals to a receiver which in turn transmits them to a device that alerts the caregiver and/or the user that the release has occurred. Disposable absorbent articles which include sensors or electric circuits inside its structure are described in international patent applications WO2012084924, WO2012084985 and WO2013091728 filed by SCA Hygiene Products; US patents US7667608, US8222476, US8264362, US 08604268, US8698641 as well as application WO2013091728 assigned to Kimberly Clark.

These types of sensors that are placed within the structure of the diaper have the significant disadvantage that may be in contact with the skin or cause discomfort for the user when triggered.

Sensors have also been developed which are not placed within the structure of the diaper. These sensors are placed outside the diaper, but remain in contact with it and may be interchangeable. This type of sensors are described in patents: US8207394, US8274393, US8440877, as well as in patent applications: WO2012166766, WO2013011391, WO2013076593, WO2013084085, US2013110061 and US2013110063, all filed by Kimberly Clark Corporation, as well as patent applications WO2012166766 and WO2012166765 of Procter & Gamble and application WO2013095230 filed by SCA Hygiene Products. These sensors have been developed to detect changes in the absorbent article, i.e., urine discharges, the volume thereof, pH or temperature changes due to the presence of urine or fecal matter, for which reason they must be in direct contact with the article, although they are not provided within the structure thereof.

The present invention refers to a system for detecting the vital signs of the user of a disposable absorbent article. The system foresees the use of a device that is in contact with the user, but not with the disposable absorbent article, although it is associated thereto. The device detects the vital signs of the user and monitors them at every moment, independently if the user has had any kind of urine and/or feces release onto the absorbent article. The device sends the signals wirelessly to a receiver, the receiver may be a mobile device, or it may send the data to a mobile device such that the caregiver of the baby or adult may check the status of the vital signs of the article user any time during the day, wherein the checking of the vital signs of the article user is activated by means of a code or trigger mechanism of a device linked to the disposable absorbent article.

**Objectives of the Invention**

The objective of the present invention is to develop a monitoring system for vital signs in babies and adults; the system is linked to the use of a disposable absorbent article.

An additional objective of the invention is that the system uses a sensor device in contact with the user, and linked to the disposable absorbent article, but not in contact thereto.

Another objective of the invention is that the device detects the vital signs and sends the signal to a receiver.

An additional objective of the invention is that the sensor is non-invasive and may be in contact with the skin of the user for long lengths of time without causing injury to the user.

Another objective of the invention is that the detected data can be checked by the caregiver of the user of a disposable absorbent article through the receiver.

A further objective of the invention is that the system outputs an alert when the detected vital sign(s) exceed certain predetermined limits.

## Brief Description of the Drawings

Figure 1 illustrates the monitoring system for vital signs of the present invention.
Figures 2a-2d shows examples of sensor devices with different frames that could be used in the monitoring system for vital signs of the present invention.
Figures 3a-3d illustrates different types of bi-dimensional codes that may be used in the monitoring system for vital signs of the present invention.
Figures 4a-4d shows examples of receiver that may be used in the monitoring system for vital signs of the present invention.

## Detailed Description of the Invention

The present invention refers to a monitoring system for vital signs of a user of a disposable absorbent article such as a disposable diaper, disposable pants, feminine sanitary towel, etc.

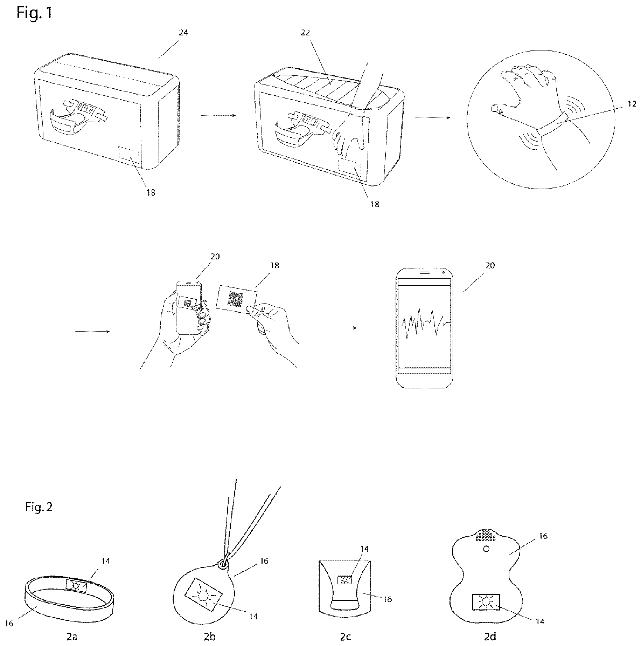

The monitoring system, shown in Figure 1, comprises:

- A sensor device (12) which is placed in contact with the user,
- A code (18) or trigger mechanism of the device,
- One or more disposable absorbent articles (22) placed inside a packaging (24),
- a receiver (20).

The sensor device (12) is designed for measuring one or more vital signs such as pulse, temperature, breathing rate, and/or blood pressure and shall be in contact with the user. This sensor device (12) is comprised by a sensor that is placed within a frame, such as a bracelet, a necklace, a self-stick patch, a clip, etc., and is placed in contact with the user.

When placing the sensor device (12) in contact with the user, it begins to detect the vital signs. For the device to send the data to the receiver and be visualized, the device shall be triggered by a code (18) or mechanism that is placed, preferably, inside the packaging (24) of the disposable absorbent articles. Once the system is triggered, the vital signs of the user can be checked on the receiver (20), which may be directly a mobile device, or any other type of receiver that in turn, is capable of sending the signal via internet to a mobile device.

The sensor device (12) comprises a sensor (14) programmed to detect pulse, blood pressure, breathing rate, and/or temperature and a frame (16) within which said sensor is placed. Figures 2a to 2d illustrate different types of frames (16) that may be used along with the device (14) to form the sensor device (12). The frame may be a bracelet (Fig. 2a), a medal (Fig. 2b), a clip (Fig. 2c), a self-stick patch (Fig. 2d), or any other device that may house the sensor and that may be placed in contact with the user. In the preferred embodiment of the invention, the frame (16) is a bracelet that may be placed on the wrist or on the ankle of the user, the frame (16) is made of a material that does not injure or irritate the skin of the user of the disposable absorbent article, even he is in contact with same for long periods of time, the material can be woven or non-woven fabric, polyurethane foam, high-density polyethylene, wood, bamboo, metal, or any other type of material suitable for such ends. Further, it is desirable for the frame material to be natural and/or renewable.

In case the frame (16) is a clip, it can be attached to any underwear of the user or to the disposable absorbent article (22), for instance, at the waist part of the article.

The sensor device may have a useful life of up to 3 years and is recharged by USB, replaceable batteries, rechargeable batteries, solar energy or any other power source.

The data detected by the sensor device (12) may be read through a receiver (20) which requires the system to be triggered by a bi-dimensional code (18) which is preferably placed inside the packaging (24) of the disposable absorbent articles (22). Once the system is triggered, the caregiver may check the detected data on the receiver. The entire system is illustrated in Fig. 1. The triggering of the sensor device has a service time, i.e., the receiver is capable of reading and displaying the data for a predetermined length of time, after which the system is automatically deactivated and the data may no longer be read on the receiver. The system may be activated again using a new bi-dimensional code (18) that will be obtained from a new packaging (24) of disposable absorbent articles (22). The time during which the system will be active (activation period) is associated with the read code, and this, in turn, is associated with the number of articles placed inside the bag or packaging (24), such that the system is programmed to be active during the time in which all the diapers contained in the packaging are used. For instance, in a packaging containing 12 articles, the system shall have an activation period of 4 days, considering that at least 3 diapers are used per day; thus, in a packaging of 40 articles, when reading the bi-dimensional code, the system will be activated during at least 14. The minimum activation period a code (18) will have is calculated based on the following formula:

```
ACTIVATION PERIOD = Integer resulting of the division of the number of articles inside the packaging/3 + 1
```

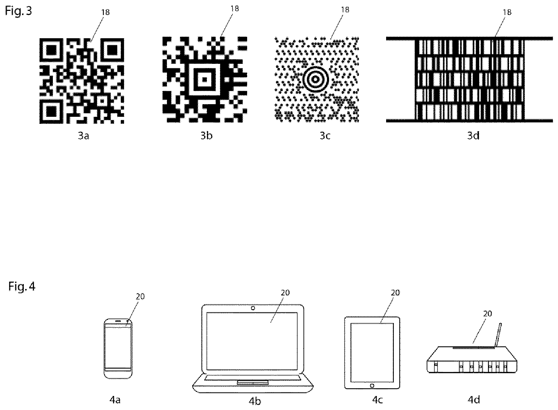

The triggering code can be any type of bi-dimensional code (18), such as bar code, QR (quick response) code, Data Matrix code, Aztec code, 49 code, Maxicode, PDF417 code, numeric, etc., as shown in Figure 3. Preferably, the code may be read using an image reader.

The code (18) is preferably placed in the inner part of the bag or packaging (24) that contains the disposable absorbent articles. In the preferred embodiments of the invention, the code (18) is printed on the inner part of the bag or packaging (24), placed on the inner part of the by a self-stick label or may be printed onto a card, label or any other object that is placed inside the packaging (24). The code (18) may also be printed on the outer part of at least one article (22) of those that are inside the packaging (24). Finally, in another embodiment of the invention, the code (18) may also be printed on the outer part of the bag or packaging (24).

Once the caregiver opens the packaging, she/he may trigger the system through the receiver (20) using an image reader or introducing the bi-dimensional code data. The receiver (20) as illustrated in Figures 4a to 4b, may be a mobile telephone, a modem, a tablet, a computer or any other type of wired or wireless receiver. Upon the triggering, the caregiver will be able to check the vital signs of the user of the sensor device (12) on the receiver (20) which shall be placed within a maximum radius of 15 linear meters from the sensor device (12). The data may only be checked during the activation period, once this ends, the sensor device (12) will continue detecting the vital signs, but the caregiver will not be able to check them until he or she triggers again the system using another bi-dimensional code which can be obtained from another packaging.

Furthermore, in case the caregiver or any other person is interested in knowing the vital signs of the user of the sensor device but is located more than the previously indicated 15 linear meters radius away from the sensor device, he may access the information of the vital signs through the internet, through a mobile or electronic device, such that the caregiver or any interested adult (the parents, for example) may check the vital signs of the baby or of the adult wherever they are and at any time of the day or night.

In one embodiment of the invention, the system will output an alarm to alert the caregiver when the vital signs exceed certain predetermined limits.

The detected data can be stored for as long as the caregiver programs and may be processed in such a way that the caregiver may check the graphics of the variation of each of the detected vital sign over time.

The monitoring system for vital signs of the present invention may be associated with any article that is placed inside a packaging, preferably associated to the care of babies or incontinent adults, for example moist towels.

While the invention has been described according to the currently preferred embodiment, it is evident that several changes or modifications thereto can be made, such changes and modifications are within the scope and spirit of the invention as defined in the appended claims.

**Claims**

1.    A monitoring system for vital signs of a user of a disposable absorbent article which comprises a sensor device, a bi-dimensional code and a receiver, wherein said system is **characterized in that** said sensor device is placed in contact with the user and the system is triggered through the reading of the bi-dimensional bar code by the receiver, such that the bi-dimensional code is placed inside the disposable absorbent article packaging.

2.    The monitoring system for vital signs of a user of a disposable absorbent article according to claim 1, further **characterized in that** said sensor device comprises a sensor programed to read at least one vital sign and a frame inside which said sensor is placed.

3.    The monitoring system for vital signs of a user of a disposable absorbent article according to claims 1 and 2, further **characterized in that** said sensor is programed to monitor the pulse of the user of the disposable absorbent article.

4.    The monitoring system for vital signs of a user of a disposable absorbent article according to claims 1 and 2, further **characterized in that** said sensor is programed to monitor the breathing rate of the user of the disposable absorbent article.

5.    The monitoring system for vital signs of a user of a disposable absorbent article according to claims 1 and 2, further **characterized in that** said sensor is programmed to monitor the temperature of the user of the disposable absorbent article.

6. The monitoring system for vital signs of a user of a disposable absorbent article according to claims 1 and 2, further **characterized in that** said sensor is programmed to monitor the blood pressure of the user of the disposable absorbent article.

7. The monitoring system for vital signs of a user of a disposable absorbent article according to claims 1 and 2, further **characterized in that** said sensor is programmed to monitor the pulse, the temperature, the breathing rate and the blood pressure of the user of the disposable absorbent article.

8. The monitoring system for vital signs of a user of a disposable absorbent article according to any of the preceding claims, further **characterized in that** the frame is a bracelet.

9. The monitoring system for vital signs of a user of a disposable absorbent article according to claims 1 to 7, further **characterized in that** said frame is a self-stick patch.

10. The monitoring system for vital signs of a user of a disposable absorbent article according to claims 1 to 7, further **characterized in that** said frame is a medal.

11. The monitoring system for vital signs of a user of a disposable absorbent article according to claims 1 to 7, further **characterized in that** said frame is a clip.

12. The monitoring system for vital signs of a user of a disposable absorbent article according to claims 1 to 8, further **characterized in that** said frame is of a renewable material.

13. The monitoring system for vital signs of a user of a disposable absorbent article according to claims 8 to 11, further **characterized in that** said frame is of a natural origin material.

14. The monitoring system for vital signs of a user of a disposable absorbent article according to claims 8 to 7, further **characterized in that** the material of the frame may be non-woven fabric, fabric, rubber, wood, metal, bamboo, polyurethane foam or high-density polyethylene.

15. The monitoring system for vital signs of a user of a disposable absorbent article according to claims 2 to 14, further **characterized in that** said sensor device is rechargeable through a USB, replaceable batteries, rechargeable batteries, or through sunlight.

16. The monitoring system for vital signs of a user of a disposable absorbent article according to claims 2 to 15, further **characterized in that** the useful life of the sensor device is of maximum 3 years.

17. The monitoring system for vital signs of a user of a disposable absorbent article according to any of the preceding claims, further **characterized in that** the bi-dimensional code may be a QR code, a Data Matrix code, an Aztec code, a 49 code, a Maxicode code, a PDF417 code or a numeric code.

18. The monitoring system for vital signs of a user of a disposable absorbent article according to claim 17, further **characterized in that** said bi-dimensional code is placed inside the disposable absorbent article packaging.

19. The monitoring system for vital signs of a user of a disposable absorbent article according to claim 17, further **characterized in that** the bi-dimensional code is printed inside the disposable absorbent article packaging.

20. The monitoring system for vital signs of a user of a disposable absorbent article according to claim 17, further **characterized in that** the bi-dimensional code is printed on any object that is placed inside the disposable absorbent article packaging.

21. The monitoring system for vital signs of a user of a disposable absorbent article according to claim 20, further **characterized in that** the bi-dimensional code is printed on a card or a label.

22. The monitoring system for vital signs of a user of a disposable absorbent article according to claim 20, further **characterized in that** the bi-dimensional code is printed on the outer part of at least one disposable absorbent article that is placed inside the packaging.

23. The monitoring system for vital signs of a user of a disposable absorbent article according to claim 17, further **characterized in that** the bi-dimensional code is printed on the outer part of the disposable absorbent articles packaging.

24. The monitoring system for vital signs of a user of a disposable absorbent article according to any of the preceding claims, further **characterized in that** the system is only active during an activation period that is determined in the bi-dimensional code.

25. The monitoring system for vital signs of a user of a disposable absorbent article according to claim 24, further **characterized in that** the minimum activation period is calculated according to the number of disposable absorbent articles inside the packaging using the following formula:

```
Minimum activation period = Integer resulting of the division of the number of articles inside the packaging/3 + 1
```

26. The monitoring system for vital signs of a user of a disposable absorbent article according to any of the preceding claims, further **characterized in that** the receiver is a cell phone with an image reader.

27. The monitoring system for vital signs of a user of a disposable absorbent article according to anyone of claims 1 to 25, further **characterized in that** the receiver is a tablet, a modem or a computer.

28. The monitoring system for vital signs of a user of a disposable absorbent article according to any of the preceding claims, further **characterized in that** the receiver is capable of reading and displaying the vital signs of the user of the article within a maximum radius of 15 meters of the sensor device.

29. The monitoring system for vital signs of a user of a disposable absorbent article according to any of the preceding claims, further **characterized in that** the receiver uploads the data of the vital signs of the user to the internet, such that they may be read by a mobile device located at any distance from the sensor device.

30. The monitoring system for vital signs of a user of a disposable absorbent article according to any of the preceding claims, further **characterized in that** it sends an alert when the detected vital sign(s) exceed certain predetermined limits.

31. A monitoring system of the vital signs of a user of an article located inside a packaging which comprises a sensor device, a bi-dimensional code and a receiver, said system is further **characterized in that** the sensor device is placed in contact with the user and the system is activated by the reading of the bi-dimensional code through the receiver, such that the bi-dimensional code is located inside the packaging.

32. The monitoring system for vital signs according to claim 31, further **characterized in that** the sensor device comprises a sensor programmed to read at least one vital sign and a frame within which said sensor is located.

33. The monitoring system for vital signs of a user of an article placed inside a packaging according to claims 31 and 32, further **characterized in that** the sensor is programmed to monitor the pulse of the user of the disposable absorbent article.

34. The monitoring system for vital signs of a user of an article placed inside a packaging according to claims 31 and 32, further **characterized in that** the sensor is programmed to monitor the breathing rate of the user of the article.

35. The monitoring system for vital signs of a user of an article placed inside a packaging according to claims 31 and 32, further **characterized in that** the sensor is programmed to monitor the temperature of the user of the article.

36. The monitoring system for vital signs of a user of an article placed inside a packaging according to claims 31 and 32, further **characterized in that** the sensor is programmed to monitor the blood pressure of the user of the article.

37. The monitoring system for vital signs of a user of an article placed inside a packaging according to claims 31 and 32, further **characterized in that** the sensor is programmed to monitor the pulse, the temperature, the breathing rate and the blood pressure of the user of the article.

38. The monitoring system for vital signs of a user of an article placed inside a packaging according to anyone of claims 31 and 32, further **characterized in that** said frame is a bracelet.

39. The monitoring system for vital signs of a user of an article placed inside a packaging according to claims 31 to 37, further **characterized in that** said frame is a self-stick patch.

40. The monitoring system for vital signs of a user of a an article placed inside a packaging according to claims 31 to 37, further **characterized in that** said frame is a medal.

41. The monitoring system for vital signs of a user of an article placed inside a packaging according to claims 31 to 37, further **characterized in that** said frame is a clip.

42. The monitoring system for vital signs of a user of an article placed inside a packaging according to claims 38 to 41, further **characterized in that** said frame is of a renewable material.

43. The monitoring system for vital signs of a user of an article placed inside a packaging according to claims 38 to 41, further **characterized in that** said frame is of a natural origin material.

44. The monitoring system for vital signs of a user of an article placed inside a packaging according to claims 38 to 41, further **characterized in that** said frame may be non-woven fabric, fabric, rubber, wood, metal, bamboo, polyurethane foam or high-density polyethylene.

45. The monitoring system for vital signs of a user of an article placed inside a packaging according to anyone of claims 32 to 44, further **characterized in that** the sensor device is rechargeable by USB, replaceable batteries, rechargeable batteries, or by sunlight.

46. The monitoring system for vital signs of a user of an article placed inside a packaging according to anyone of claims 32 to 45, further **characterized in that** the useful life of the sensor device is of maximum 3 years.

47. The monitoring system for vital signs of a user of an article placed inside a packaging according to anyone of claims 38 to 46, further **characterized in that** the bi-dimensional code may be a QR code, a Data Matrix code, an Aztec code, a 49 code, a Maxicode code, a PDF417 code or a numeric code.

48. The monitoring system for vital signs of a user of an article placed inside a packaging according to claim 47, further **characterized in that** the bi-dimensional code is placed inside the article packaging.

49. The monitoring system for vital signs of a user of an article placed inside a packaging according to claim 47, further **characterized in that** the bi-dimensional code is printed inside the article packaging.

50. The monitoring system for vital signs of a user of an article placed inside a packaging according to claim 47, further **characterized in that** the bi-dimensional code is printed on any object that is placed inside the packaging.

51. The monitoring system for vital signs of a user of an article placed inside a packaging according to claim 50, further **characterized in that** the bi-dimensional code is printed onto a card or label.

52. The monitoring system for vital signs of a user of an article placed inside a packaging according to claim 50, further **characterized in that** the bi-dimensional code is printed on the outer part of at least one of the articles placed inside the packaging.

53. The monitoring system for vital signs of a user of an article placed inside a packaging according to claim 47, further **characterized in that** the bi-dimensional code is printed onto the outer part of the packaging.

54. The monitoring system for vital signs of a user of an article placed inside a packaging according to anyone of claims 31 to 53, further **characterized in that** the system is only active during an activation period which is determined in the bi-dimensional code.

55. The monitoring system for vital signs of a user of an article placed inside a packaging according to anyone of claims 31 to 54, further **characterized in that** the receiver is a cell phone with an image reader.

56. The monitoring system for vital signs of a user of an article placed inside a packaging according to anyone of claims 31 to 54, further **characterized in that** the receiver is a tablet, a modem or a computer.

57. The monitoring system for vital signs of a user of an article placed inside a packaging according to anyone of claims 31 to 56, further **characterized in that** the receiver is capable of reading and displaying the vital signs of the user of the article within a maximum radius of 15 meters of the sensor device.

58. The monitoring system for vital signs of a user of an article placed inside a packaging according to anyone of claims 31 to 57, further **characterized in that** the receiver uploads the vital signs data of the user to the internet, such that said vital signs may be read by a mobile device located at any distance from the sensor device.

59. The monitoring system for vital signs of a user of an article placed inside a packaging according to anyone of claims 31 to 58, further **characterized in that** it sends an alert when the detected vital sign(s) exceed certain predetermined limits.

EP 3 184 039 A1

Fig. 1

Fig. 2

2a     2b     2c     2d

Fig. 3

3a    3b    3c    3d

Fig. 4

4a    4b    4c    4d

## INTERNATIONAL SEARCH REPORT

| International application No. |
| --- |
| PCT/IB2015/055526 |

**A. CLASSIFICATION OF SUBJECT MATTER**

*A61B5/02* (2006.01)
*H04W4/00* (2009.01)
According to International Patent Classification (IPC) or to both national classification and IPC

**B. FIELDS SEARCHED**

Minimum documentation searched (classification system followed by classification symbols)
A61B, H04W

Documentation searched other than minimum documentation to the extent that such documents are included in the fields searched

Electronic data base consulted during the international search (name of data base and, where practicable, search terms used)

EPODOC, INVENES, WPI, NPL, INSPEC, BIOSIS, MEDLINE

**C. DOCUMENTS CONSIDERED TO BE RELEVANT**

| Category* | Citation of document, with indication, where appropriate, of the relevant passages | Relevant to claim No. |
| --- | --- | --- |
| X | US 2014051946 A1 (ARNE ET AL.) 20.02.2014, paragraphs 2, 7, 30, 32, 34, 37, 77, 79, 116, 117 | 1-19, 21-25, 27-60 |
| X | WO 2014095644 (SANOFI-AVENTIS DEUTSCHLAND GMBH) 26.06.2014, page 1, lines 3 - 4; page 2, lines 24 - 27; page 3, lines 1 - 8; page 6, line 22 - page 7, line 19; page 9, line 29 - page 10, line 14; page 14, lines 19 - 31; | 1-19, 21-25, 27-60 |
| A | US 2013131618 A1 (ABRAHAM ET AL.) 23.05.2013, abstract; paragraph 47 | 1, 32 |
| A | US 2013110061 A1 (ABRAHAM ET AL.) 02.05.2013, abstract; paragraph 13 | 1, 32 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 17/11/2015 | **(17/11/2015)** |

| Name and mailing address of the ISA/ | Authorized officer |
| --- | --- |
| OFICINA ESPAÑOLA DE PATENTES Y MARCAS Paseo de la Castellana, 75 - 28071 Madrid (España) Facsimile No.: 91 349 53 04 | A. Cárdenas Villar Telephone No. 91 3495393 |

Form PCT/ISA/210 (second sheet) (January 2015)

## INTERNATIONAL SEARCH REPORT

| International application No. |
|---|
| PCT/IB2015/055526 |

**C (continuation).** DOCUMENTS CONSIDERED TO BE RELEVANT

| Category * | Citation of documents, with indication, where appropriate, of the relevant passages | Relevant to claim No. |
|---|---|---|
| A | US 2009270743 A1 (DUGAN ET AL.) 29.10.2009, abstract; paragraph 11; claims 1 - 9 | 1-8, 14, 32-39, 45 |
| A | US 2010274104 A1 (KHAN) 28.10.2010, claims 1 - 21 | 1-7, 32-38 |
| A | US 2013178753 A1 (WU) 11.07.2013, claims 1 - 4 | 1-3, 8, 32-34, 39 |
| A | WO 2013131275 A1 (SHENZHEN BELTER HEALTH MEASUREMENT AND ANALYSIS TECHNOLOGY CO., LTD.) 12.09.2013, abstract | 1-7, 32-38 |

Form PCT/ISA/210 (continuation of second sheet) (January 2015)

## INTERNATIONAL SEARCH REPORT

Information on patent family members

| International application No. |
|---|
| PCT/IB2015/055526 |

| Patent document cited in the search report | Publication date | Patent family member(s) | Publication date |
|---|---|---|---|
| US2014051946 A1 | 20.02.2014 | TW201501692 A | 16.01.2015 |
| | | JP2014208301 A | 06.11.2014 |
| | | WO2014151925 A1 | 25.09.2014 |
| | | US2014236077 A1 | 21.08.2014 |
| | | US9149577 B2 | 06.10.2015 |
| | | HK1162903 A1 | 27.06.2014 |
| | | US2014009262 A1 | 09.01.2014 |
| | | TW201320961 A | 01.06.2013 |
| | | JP2013010029 A | 17.01.2013 |
| | | JP5591298B B2 | 17.09.2014 |
| | | CN102885615 A | 23.01.2013 |
| | | IL221790 A | 31.08.2015 |
| | | CA2792224 A1 | 01.07.2010 |
| | | JP2012511969 A | 31.05.2012 |
| | | JP5143290B B2 | 13.02.2013 |
| | | US2012101430 A1 | 26.04.2012 |
| | | US8545436 B2 | 01.10.2013 |
| | | SG172165 A1 | 28.07.2011 |
| | | CN102316795 A | 11.01.2012 |
| | | CN102316795B B | 30.10.2013 |
| | | AU2009330321 A1 | 14.07.2011 |
| | | AU2009330321B B2 | 07.03.2013 |
| | | KR20110094147 A | 19.08.2011 |
| | | KR101146555B B1 | 25.05.2012 |
| | | TW201034625 A | 01.10.2010 |
| | | TWI424832B B | 01.02.2014 |
| | | IL213491 A | 28.11.2013 |
| | | CA2747156 A1 | 01.07.2010 |
| | | CA2747156 C | 22.10.2013 |
| | | US2010312188 A1 | 09.12.2010 |
| | | US8114021 B2 | 14.02.2012 |
| | | WO2010075115 A2 | 01.07.2010 |
| | | WO2010075115 A3 | 23.09.2010 |
| | | EP2358271 A2 | 24.08.2011 |
| | | EP2358271 A4 | 18.12.2013 |
| WO2014095644 A1 | 26.06.2014 | US2015320314 A1 | 12.11.2015 |
| | | CN104837400 A | 12.08.2015 |
| | | EP2941180 A1 | 11.11.2015 |
| US2013131618 A1 | 23.05.2013 | WO2013076593 A1 | 30.05.2013 |
| US2013110061 A1 | 02.05.2013 | MX2014004530 A | 01.08.2014 |
| | | KR20140084056 A | 04.07.2014 |
| | | CN103890550 A | 25.06.2014 |
| | | AU2012328092 A1 | 10.04.2014 |
| | | AU2012328092B B2 | 15.10.2015 |
| | | US9119748 B2 | 01.09.2015 |
| | | EP2771649 A1 | 03.09.2014 |
| | | EP2771649 A4 | 01.07.2015 |

Form PCT/ISA/210 (patent family annex) (January 2015)

13

## INTERNATIONAL SEARCH REPORT

Information on patent family members

International application No.

PCT/IB2015/055526

| Patent document cited in the search report | Publication date | Patent family member(s) | Publication date |
|---|---|---|---|
| | | WO2013061181 A1 | 02.05.2013 |
| US2009270743 A1 | 29.10.2009 | US2015151198 A1 | 04.06.2015 |
| | | US2015038204 A1 | 05.02.2015 |
| | | US2010033303 A1 | 11.02.2010 |
| | | US8976007 B2 | 10.03.2015 |
| US2010274104 A1 | 28.10.2010 | WO2009049104 A1 | 16.04.2009 |
| US2013178753 A1 | 11.07.2013 | NONE | |
| WO2013131275 A1 | 12.09.2013 | NONE | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012084924 A
- WO 2012084985 A
- WO 2013091728 A
- US 7667608 B
- US 8222476 B
- US 8264362 B
- US 08604268 B
- US 8698641 B
- US 8207394 B
- US 8274393 B
- US 8440877 B
- WO 2012166766 A
- WO 2013011391 A
- WO 2013076593 A
- WO 2013084085 A
- US 2013110061 A
- US 2013110063 A
- WO 2012166765 A
- WO 2013095230 A